(19) Europäisches Patentamt
European Patent Office
Office européen des brevets

(11) Numéro de publication: **0 442 258 A2**

(12) **DEMANDE DE BREVET EUROPEEN**

(21) Numéro de dépôt: **91100222.8**

(51) Int. Cl.5: **C07C 11/21**, C07C 1/30

(22) Date de dépôt: **09.01.91**

(30) Priorité: **15.02.90 CH 487/90**

(43) Date de publication de la demande: **21.08.91 Bulletin 91/34**

(84) Etats contractants désignés: **CH DE FR GB LI NL**

(71) Demandeur: **FIRMENICH SA**
**1, route des Jeunes**
**CH-1211 Genève 8(CH)**

(72) Inventeur: **Gaudin, Jean-Marc**
**120,rue du Parc**
**F-74100 Annemasse(FR)**
Inventeur: **Morel, Cédric**
**Chemin du Velours, 18A**
**CH-1231 Conches(CH)**

(74) Mandataire: **Salvadori, Giuseppe, Dr.**
**c/o Firmenich S.A. Case Postale 239**
**CH-1211 Genève 8(CH)**

(54) **Procédé pour la préparation d'une oléfine polyinsaturée.**

(57) L'undéca-1,3,5-triène sous forme d'un mélange isomérique ayant un contenu prépondérant en l'isomère undéca-1,3E,5Z-triène est obtenu par addition du 1,4-dichloro-but-2-ène, du 1,2-dichloro-but-3-ène ou tout mélange desdits composés à un dérivé organo-métallique de formule

$$H_{11}C_5-\equiv-Y \qquad (I)$$

dans laquelle Y désigne un métal alcalin ou un groupe MgX, X représentant un atome d'halogène, suivie de la réduction de la triple liaison acétylénique du composé obtenu et traitement par un agent basique.

Utilisation du produit ainsi obtenu à titre d'agent parfumant.

EP 0 442 258 A2

La présente invention a trait au domaine de l'industrie de la parfumerie. En particulier, elle a pour objet un procédé pour la préparation d'un composé oléfinique polyinsaturé, l'undéca-1,3,5-triène, ingrédient parfumant fort apprécié.

Depuis sa découverte [voir : Chrétien-Bessière et al., Bull. Soc. Chim. Fr. 1967, 97], nombreuses ont été les voies de synthèse proposées pour sa préparation [voir par exemple : brevet FR 74 19580 ; brevet AS 68 01077 ; demande de brevet EP 203 615 ; F. Naef et al., Helv. Chim. Acta 1975, 58, 1016 ; V. Ratovelomanana et al., Bull. Soc. Chim. Fr. 1987, 174 ; Recherches 1967, 16, 5 ; W. Boland et al., Helv. Chim. Acta 1987, 70, 1025 ; E. Block et al., J. Am. Chem. Soc. 1986, 108, 4568]. Certaines parmi les méthodes proposées ont trouvé une application industrielle et l'undéca-1,3,5-triène est à présent commercialisé sous des dénominations diverses. Caractérisé par la présence dans sa molécule de trois doubles liaisons éthyléniques, l'undéca-1,3,5-triène peut se présenter sous différentes formes isomériques dont les proportions respectives dans le produit final déterminent sa qualité olfactive. Or, nous avons pu constater qu'aucun des procédés décrits dans l'art antérieur pouvait tout à la fois satisfaire aux exigences d'économie, de sécurité ou de respect de l'environnement et offrir un produit de qualité irréprochable, supérieure à celle du produit présentement offert sur le marché.

Le procédé de l'invention présente l'avantage de permettre l'obtention d'undéca-1,3,5-triène sous forme d'un mélange isomérique dont le contenu en l'isomère undéca-1,3E,5Z-triène est prépondérant. Or c'est précisément cet isomère qui présente au mieux les propriétés odorantes les plus caractéristiques ; sa présence anoblit donc les caractères du mélange.

Le procédé de l'invention est caractérisé en ce qu'on

a. additionne du 1,4-dichloro-but-2-ène, du 1,2-dichloro-but-3-ène ou tout mélange desdits composés à un dérivé organo-métallique de formule

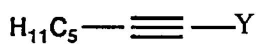

(I)

dans laquelle Y désigne un métal alcalin ou un groupe MgX, X représentant un atome d'halogène, pour fournir le chlorure d'undéc-2-én-5-yn-1-yle,

b. réduit la triple liaison acétylénique dudit composé au moyen d'une hydrogénation catalytique pour fournir le chlorure d'undéca-2,5-dién-1-yle, et

c. traite le produit ainsi obtenu avec un agent basique.

Le procédé défini ci-dessus peut être illustré à l'aide du schéma réactionnel suivant.

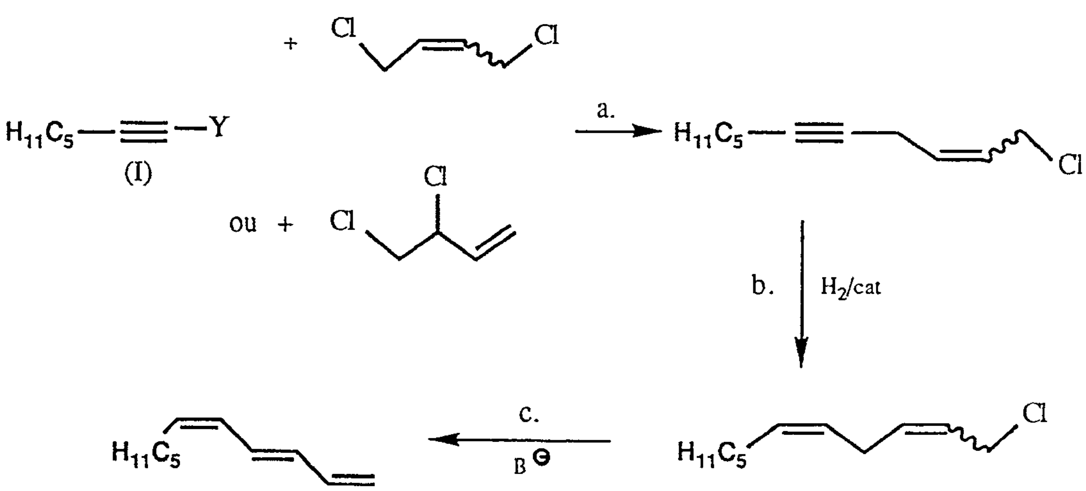


A titre de composé organo-métallique de formule (I) on peut employer des dérivés lithiés ou des magnésiens. On utilise ainsi l'heptyne-lithium ou un magnésien par exemple résultant de la réaction de l'hept-1-yne avec un halogénure d'alkyl-magnésium, par exemple le chlorure de méthyl magnésium.

L'étape d'addition a. s'effectue de préférence en présence d'un catalyseur à base de cuivre. Il s'est avéré en effet que les rendements les meilleurs pouvaient être obtenus en effectuant la réaction en présence de certains halogénures de cuivre, par exemple le chlorure, bromure ou iodure de cuivre$^{I}$, ou encore le chlorure de $Cu^{II}$. D'autres sels de cuivre peuvent également convenir. Il s'agit par exemple de

l'acétylacétonate de $Cu^{II}$ ou de l'acétate de $Cu^{II}$.

Quoique les proportions respectives des réactifs n'aient pas une influence critique, nous avons observé que l'emploi d'un excès de 1,4-dichlorobutène par rapport à l'hept-1-yne servait à diminuer considérablement le déroulement de réactions secondaires. Dans la pratique, nous avons pu déterminer que de bons rendements en produit final pouvaient être obtenus par l'emploi d'environ deux équivalents de 1,4-dichlorobutène pour un équivalent d'hept-1-yne.

L'étape suivante de réduction de la triple liaison acétylénique du chlorure d'undéc-2-én-5-yn-1-yle obtenu s'effectue dans des conditions de réactions conventionnelles [voir H.O. House, Modern Synthetic Reactions, W.A. Benjamin, Inc. (1972)]. La réaction est conduite par hydrogénation catalytique à pression atmosphérique en présence de catalyseurs au palladium partiellement empoisonnés avec de l'acétate de plomb (de type Lindlar).

La dernière étape du procédé, qui consiste en le traitement du chlorure d'undéca-2,5-dién-1-yle obtenu avec un agent basique, s'effectue de préférence en utilisant comme agent basique un alcoolate dérivé d'un alcool aliphatique inférieur, par exemple un méthylate, éthylate, iso-propylate, butylate ou encore tert-butylate de métal alcalin. Un hydroxyde de métal alcalin, tel l'hydroxyde de potassium, notamment en solution dans le tert-butanol, le toluène, le polyéthylène glycol ou le diméthylsulfoxyde, conviennent parfaitement à cette opération. Pour des raisons d'ordre pratique et économique, on utilise de préférence l'hydroxyde de potassium dans le tert-butanol ou le tert-butylate de potassium.

Comme indiqué plus haut, l'undécatriène ainsi obtenu se présente sous forme d'un mélange isomérique dans lequel toutefois le contenu en l'isomère 3E,5Z est prépondérante, sa proportion relative vis-à-vis de son isomère undéca-2Z-4Z,6E-triène est, dans la plupart des essais effectués, supérieure à 75% en poids.

Le mélange isomérique ainsi obtenu présente une qualité olfactive excellente et peut de ce fait être employé directement en parfumerie sans purification ultérieure. Toutefois, si cela devait s'avérer nécessaire, l'isomère 3E,5Z peut être séparé du mélange obtenu à l'aide des techniques de purification usuelles, par exemple par chromatographie préparative en phase gazeuse ou par simple distillation fractionnée.

Le procédé de l'invention est illustré de manière plus détaillée par l'exemple suivant dans lequel les températures sont indiquées en degrés centigrades et les abréviations ont le sens usuel dans l'art.

Exemple

a. 144g de magnésium en copeaux dans 310 g de tétrahydrofurane (THF) ont été chauffés à 45° tandis qu'on y ajoute quelque peu de 1,4-dibromoéthane tout en faisant passer dans le mélange un léger courant de chlorométhane. Une fois la réaction démarrée, on a ajouté 1 kg de THF et 330 g de chlorométhane. L'adjonction de ce dernier se fait de façon à maintenir la température à une valeur comprise entre 40 et 45°. Après réaction complète du magnésium, le mélange a été chauffé à reflux pendant 90 minutes sous atmosphère d'azote. Après refroidissement, 556,8 g de hept-1-yne ont été ajoutés au mélange et le tout a été ensuite maintenu sous agitation et sous azote à 25° pendant une nuit. L'organo-magnésien ainsi préparé a été ajouté en 35 minutes à un mélange de 1,450 kg de 1,4-dichlorobutène et 5,6 g de chlorure de cuivre. Une fois la réaction terminée (45 minutes), le mélange de réaction est versé sur un mélange d'acide acétique et eau (360 g d'acide acétique glacial dans 1,800 kg d'eau). Après séparation la phase organique a été lavée à l'eau, puis avec une solution aqueuse à 20% d'acétate de sodium et ensuite avec une solution aqueuse saturée de NaCl. L'évaporation du THF a fourni un résidu qui par distillation a fourni 1 kg de produit brut contenant à raison de 58% le chlorure d'undéc-2E-én-5-yn-1-yle (Eb. 105-108° /1,33 x $10^2$ Pa).

b. 564,5 g du chlorure d'undéc-2E-én-5-yn-1-yle en mélange avec 15,5 g de palladium du type de Lindlar dans 2,7kg d'éther de pétrole 50-70 ont été soumis à hydrogénation. Après neuf heures le mélange a été filtré, lavé avec une solution aqueuse saturée d'acétate de sodium et avec une solution aqueuse saturée de NaCl. Le filtrat clair a été concentré par évaporation de l'éther de pétrole et le résidu a été distillé. On a ainsi obtenu une fraction ayant Eb. 103-107°/12 x $10^2$ Pa constituée par le chlorure d'undéca-2,5-dién-1-yle.

c. 421 g de chlorure d'undéca-2,5-dién-1-yle ont été ajoutés en 10-15 min à un mélange de 316 g de KOH dans 887g de tert-butanol. La température du mélange a été maintenue à environ 50-75° par refroidissement extérieur. Après 1 h environ, on a séparé la phase solide de la phase liquide et le tert-butanol a été évaporé. Le produit brut ainsi obtenu a été extrait avec de l'éther de pétrole 50-70 tandis que la phase solide est dissoute dans l'eau et extraite à l'éther de pétrole 50-70. Après avoir été réunies les deux phases éthérées ont été lavées à l'eau, puis avec une solution aqueuse saturée de NaCl jusqu'à neutralité. On a ainsi obtenu une fraction ayant Eb. 80-90° / 13,3 x $10^2$ Pa contenant une proportion de 66,5% en poids d'undéca-3E,5Z-triène accompagné d'environ 23% d'undéca-2Z-4Z,6E-triène.

En remplaçant dans l'étape a. décrite plus haut le 1,4-dichloro-butène par son isomère 1,2-dichloro-but-3-ène on a obtenu le chlorure d'undéc-2E-én-5-yn-1-yle avec un rendement de 68,5%. Des résultats tout à fait similaires ont été observés lorsqu'on emploie comme produit de départ un mélange de 1,4-dichloro-but-2-ène et 1,2-dichloro-but-3-ène.

**Revendications**

**1.** Procédé pour la préparation d'undéca-1,3,5-triène sous forme d'un mélange isomérique dont le contenu en l'isomère undéca-1,3E,5Z-triène est prépondérant, caractérisé en ce qu'on

a. additionne du 1,4-dichloro-but-2-ène, du 1,2-dichloro-but-3-ène ou tout mélange desdits composés à un dérivé organo-métallique de formule

$$H_{11}C_5 - \equiv - Y \qquad (I)$$

dans laquelle Y désigne un métal alcalin ou un groupe MgX, X représentant un atome d'halogène, pour fournir le chlorure d'undéc-2-én-5-yn-1-yle,

b. réduit la triple liaison acétylénique dudit composé au moyen d'une hydrogénation catalytique pour fournir le chlorure d'undéca-2,5-dién-1-yle,et

c. traite le produit ainsi obtenu avec un agent basique.

**2.** Procédé suivant la revendication 1, caractérisé en ce que le chlorure d'undéca-2,5-dién-1-yle se présente sous sa forme isomérique de configuration 2Z,5Z et que l'on obtient par traitement dudit composé avec un agent basique l'undéca-1,3E,5Z-triène pratiquement pur.

**3.** Procédé suivant la revendication 1, caractérisé en ce que le chlorure d'undéca-2,5-dién-1-yle se présente sous forme d'un mélange d'isomères de configuration 2Z,5Z et 2E,5Z et que l'on obtient par traitement dudit composé avec un agent basique l'undéca-1,3E,5Z-triène accompagné de quantités inférieures d'undéca-2Z,4Z,6E-triène.

**4.** Procédé suivant l'une quelconque des revendications 1 à 3, caractérisé en ce qu'on utilise à titre d'agent basique un hydroxyde, un alcoolate ou un hydrure d'un métal alcalin, ou le diazabicycloundécène.

**5.** Procédé suivant la revendication 4, caractérisé en ce qu'on utilise comme agent basique le tert-butylate de potassium.

**6.** Procédé suivant la revendication 4, caractérisé en ce qu'on utilise comme agent basique l'hydroxyde de potassium et qu'on effectue la réaction dans le tert-butanol.

**7.** Utilisation d'undéca-1,3,5-triène, tel qu'obtenu selon le procédé de la revendication 1, à titre d'agent parfumant.